# EUROPEAN PATENT SPECIFICATION

(11) **EP 3 127 469 B1**
(45) Date of publication and mention of the grant of the patent: **04.09.2019**
(21) Application number: 15772917.9
(22) Date of filing: 04.03.2015
(51) Int. Cl.: A61B 1/04, A61B 1/06, G02B 23/24, G02B 23/26, A61B 1/00, A61B 1/07

(54) **ENDOSCOPE SYSTEM**
ENDOSKOPSYSTEM
SYSTÈME D'ENDOSCOPE

(30) Priority: 31.03.2014 JP 2014074280; 21.08.2014 JP 2014168449
(43) Date of publication of application: 08.02.2017
(73) Proprietor: Fujifilm Corporation, Minato-ku Tokyo 106-8620 (JP)
(72) Inventor: KURAMOTO, Masayuki, Ashigarakami-gun Kanagawa 258-8538 (JP)
(74) Representative: Klunker IP Patentanwälte PartG mbB
(86) International application number: PCT/JP2015/056359
(87) International publication number: WO 2015/151703

(56) References cited:
- EP-A1- 2 283 769
- EP-A1- 2 452 610
- WO-A1-2013/054817
- WO-A1-2013/111623
- JP-A- 2007 244 681
- JP-A- 2011 010 998
- JP-A- 2011 036 361
- JP-A- 2013 000 176

## Description

### Field of the Invention

The present invention relates to an endoscope system that illuminates an observation target using a semiconductor light source of a plurality of colors such as a blue LED, a green XLED, and a red LED.

### Description of the Related Art

In the medical field, diagnosis using an endoscope system comprising a light source device, an endoscope, and a processor device has been widely performed. A semiconductor light source of a plurality of colors obtained by combining a blue LED, a green LED, and a red LED may be used as the light source device, in addition to a broadband light source such as a xenon lamp or a white light emitting diode (LED). In a case in which such a semiconductor light source of a plurality of colors is used, light of a plurality of colors emitted from semiconductor light sources of the respective colors is combined to generate illumination light for illuminating an observation target in a body.

In a case in which light of a plurality of colors is combined to generate illumination light, the amount of light of each color can be independently adjusted and a color tone of the illumination light can be flexibly changed. Accordingly, color balance processing such as white balance processing can be performed not only on a processor device side, but also on a light source device side (see, for example, Patent Document 1). For example, in a case in which the white balance is performed on the light source device side without performing signal processing such as gain processing on the processor device side, noise caused by the gain processing or the like can be reduced.

### PRIOR ART DOCUMENTS

### Patent Documents

Patent Document 1: JP2002-122794A EP 2 452 610 A1 teaches a lighting device for an endoscope obtaining illumination light by using light emitted from a plurality of light sources.

### SUMMARY OF THE INVENTION

### Problems to Be Solved by The Invention

An image obtained by imaging an observation target such as a stomach or an esophagus in a body using an endoscope is generally reddish. This is because the observation target is greatly affected by absorption characteristics of hemoglobin, and a signal level (for example, an average value of a pixel value) of an R image signal in an RGB image signal obtained by imaging the observation target is higher than signal levels of a G image signal and a B image signal.

Further, in the observation using the endoscope, a coloring agent such as a pyoktanin coloring agent may be sprayed onto the observation target according to a purpose of diagnosis, and a residue or a residual liquid such as bilirubin may be detected on the observation target. Since the coloring agent, or the residue or residual liquid has light absorption characteristics, an observation target in which there is the coloring agent or the like is affected by not only the absorption characteristics of the hemoglobin, but also absorption characteristics of substances such as the coloring agent, or the residue or residual liquid other than the hemoglobin. In this case, a relationship between the signal levels of the RGB image signal is changed due to presence of a coloring agent or the like.

For example, in a case in which a pyoktanin coloring agent is sprayed for the purpose of observing a pit pattern on an observation target, a green component in illumination light is absorbed by the pyoktanin coloring agent. In this case, a signal level of a G image signal in a case in which the pyoktanin coloring agent is sprayed is lower than a signal level of a G image signal in a case in which there is only hemoglobin. The pit pattern is clarified due to the spraying of the pyoktanin coloring agent even in the event that the signal level of the G image signal is lowered in this way, but it is necessary to increase the signal level of the G image signal that has been lowered by the light absorption due to the pyoktanin coloring agent in order to further clarify the pit pattern. Therefore, it has been required to emphasize a slight difference in color tone and improve visibility of a part of interest on an observation target even in a case in which absorption substances other than hemoglobin are mixed.

Applying of Patent Document 1 is considered to solve the problem, but a light emission ratio of light of respective colors after white balance is set is fixed in Patent Document 1. Therefore, even in a case in which light absorption substances such as a pyoktanin coloring agent other than hemoglobin are mixed with an observation target in a body and a signal level of an image signal of a specific color is lowered, the lowered signal level of the image signal of the specific color cannot be increased in a method of Patent Document 1.

An object of the present invention is to provide an endoscope system and a method of operating the same capable of emphasizing a slight difference between color tones and improving visibility of a part of interest on an observation target even in a case in which absorption substances such as a pyoktanin coloring agent other than hemoglobin are mixed.

### Means for Solving the Problems

To achieve the above object, an endoscope system of the present invention comprises: a light source unit that emits red light, green light, and blue light at a first light emission ratio; an image pick-up sensor that images an observation target in a body, and outputs a color image signal including an image signal of a first color, an image signal of a second color, and an image signal of a third color, the image pick-up sensor imaging the observation target in the body illuminated by the red light, the green light, and the blue light at the first light emission ratio and outputting a first color image signal; and a control unit that changes a light emission ratio of the red light, the green light, and the blue light from the first light emission ratio to a second light emission ratio, and controls the amount of light of at least one color among the red light, the green light, and the blue light so that a signal level of the image signal of the second color or the image signal of the third color in the first color image signal matches a signal level of the image signal of the first color.

The image pick-up sensor images the observation target illuminated by the red light, the green light, and the blue light at the second light emission ratio and output a second color image signal. It is preferable that the image signal of the first color is a red image signal, the image signal of the second color is a green image signal, and the image signal of the third color is a blue image signal, the first color image signal is a first red image signal, a first green image signal, or a first blue image signal, and the second color image signal is a second red image signal, a second green image signal, or a second blue image signal.

It is preferable for the endoscope system to further comprise: a first correction unit that corrects the second green image signal or the second blue image signal so that the signal level of the second green image signal or the second blue image signal matches a signal level of the second red image signal in a case in which the signal level of the second green image signal or the second blue image signal is outside a specific range. It is preferable for the endoscope system to further comprise: a signal level storage unit that stores a signal level of the first green image signal or the first blue image signal; and a second correction unit that corrects the second green image signal so that the signal level of the second green image signal becomes a signal level of the first green image signal, or corrects the second blue image signal so that a signal level of the second blue image signal becomes a signal level of the first blue image signal.

It is preferable for the endoscope system to further comprise a display unit that displays a first special image on the basis of the second red image signal, the second green image signal, and the second blue image signal, and displays a second special image on the basis of the second red image signal, the second green image signal after correction, and the second blue image signal after correction.

It is preferable that the endoscope system further comprises an average value calculation unit that calculates an average value of the pixel values of the first red image signal, an average value of the pixel values of the first green image signal, and an average value of the pixel values of the first blue image signal on the basis of the first red image signal, the first green image signal, and the first blue image signal, and the control unit controls the amount of light of at least one color among the red light, the green light, and the blue light so that the average value of the pixel values of the first green image signal or the first blue image signal matches the average value of the pixel values of the first red image signal. It is preferable for a pyoktanin coloring agent to be included in the observation target.

It is preferable that the endoscope system further comprises a target value setting unit that sets a first target value from the signal level of the image signal of the first color in the first color image signal, and the control unit changes the light emission ratio of the red light, the green light, and the blue light from the first light emission ratio to the third light emission ratio, instead of changing the light emission ratio of the red light, the green light, and the blue light from the first light emission ratio to the second light emission ratio, and controls the amount of light of at least one color among the red light, the green light, and the blue light so that respective signal levels of the image signal of the first color, the image signal of the second color, and the image signal of the third color in the first color image signal match the first target value.

It is preferable that the endoscope system further comprises a target value setting unit that sets a first target value from the signal level of the image signal of the first color in the first color image signal, sets a second target value from the signal level of the image signal of the second color in the first color image signal, and sets a third target value from the signal level of the image signal of the third color in the first color image signal, and the control unit changes the light emission ratio of the red light, the green light, and the blue light from the first light emission ratio to the fourth light emission ratio, instead of changing the light emission ratio of the red light, the green light, and the blue light from the first light emission ratio to the second light emission ratio, and controls the amount of light of at least one color among the red light, the green light, and the blue light so that the signal level of the image signal of the first color in the first color image signal matches the first target value, the signal level of the image signal of the second color in the first color image signal matches the second target value, and the signal level of the image signal of the third color in the first color image signal matches the third target value. It is preferable for indigo carmine to be contained in the observation target. It is preferable for iodine to be contained in the observation target.

It is preferable that the observation target includes at least hemoglobin, and the signal level of the image signal of the first color is higher than the signal level of the image signal of the second color and the image signal of the third color in the first color image signal.

A method of operating an endoscope system of the present invention comprises: a step in which a light source unit emits red light, green light, and blue light at a first light emission ratio; a step in which an image pick-up sensor that images an observation target in a body and outputs a color image signal including an image signal of a first color, an image signal of a second color, and an image signal of a third color images the observation target in the body illuminated by the red light, the green light, and the blue light at the first light emission ratio and outputs a first color image signal; and a step in which a control unit changes a light emission ratio of the red light, the green light, and the blue light from the first light emission ratio to a second light emission ratio, and controls the amount of light of at least one color among the red light, the green light, and the blue light so that a signal level of the image signal of the second color or the image signal of the third color in the first color image signal matches a signal level of the image signal of the first color.

### Effect of the Invention

According to the present invention, which is defined in independent claim 1 and the dependent claims 2 - 8, it is possible to emphasize a slight difference in color tone and improve visibility of a part of interest on an observation target even in a case in which absorption substances such as a pyoktanin coloring agent other than hemoglobin are mixed.

### BRIEF DESCRIPTION OF THE DRAWINGS

Fig. 1 is an external view of an endoscope system.
Fig. 2 is a block diagram illustrating a function of the endoscope system.
Fig. 3 is a graph illustrating a light emission spectrum of violet light V, blue light B, green light G, and red light R.
Fig. 4A is a graph illustrating a histogram of a first RGB image signal in a case in which a pyoktanin coloring agent is sprayed.
Fig. 4B is a graph illustrating a histogram of a second RGB image signal in a case in which a pyoktanin coloring agent is sprayed.
Fig. 5 is a block diagram illustrating a function of a special image processing unit.
Fig. 6A is a graph illustrating a histogram of a second RGB image signal in a case in which a pyoktanin coloring agent is sprayed, and is a graph in a case in which average values of pixel values of a second G image signal and a second B image signal is within a specific range.
Fig. 6B is a graph illustrating a histogram of a second RGB image signal in a case in which a pyoktanin coloring agent is sprayed, and is a graph in a case in which average values of pixel values of a second G image signal and a second B image signal is outside a specific range.
Fig. 7 is an image diagram illustrating a first special image in a case in which a pyoktanin coloring agent is sprayed.
Fig. 8 is an image diagram illustrating a second special image.
Fig. 9 is an image diagram illustrating a first special image and a second special image that are simultaneously displayed.
Fig. 10 is a flowchart illustrating a sequence of flow of the present invention.
Fig. 11 is a graph illustrating an emission spectrum of violet light V, blue light B, green light G, and red light R different from Fig. 3.
Fig. 12 is a schematic diagram of a capsule endoscope.
Fig. 13 is an illustrative diagram illustrating a 10% peak value in an R image signal.
Fig. 14 is a graph illustrating a histogram of a first RGB image signal in a case in which indigo carmine is sprayed.
Fig. 15 is an image diagram illustrating a first special image in a case in which indigo carmine is sprayed.
Fig. 16 is a graph illustrating a histogram of a second RGB image signal in a case in which indigo carmine is sprayed.
Fig. 17 is a graph illustrating a histogram of a first RGB image signal in a case in which iodine is sprayed.
Fig. 18 is an image diagram illustrating a first special image in a case in which iodine is sprayed.
Fig. 19 is a graph illustrating a histogram of a second RGB image signal in a case in which iodine is sprayed.

### DESCRIPTION OF THE PREFERRED EMBODIMENTS

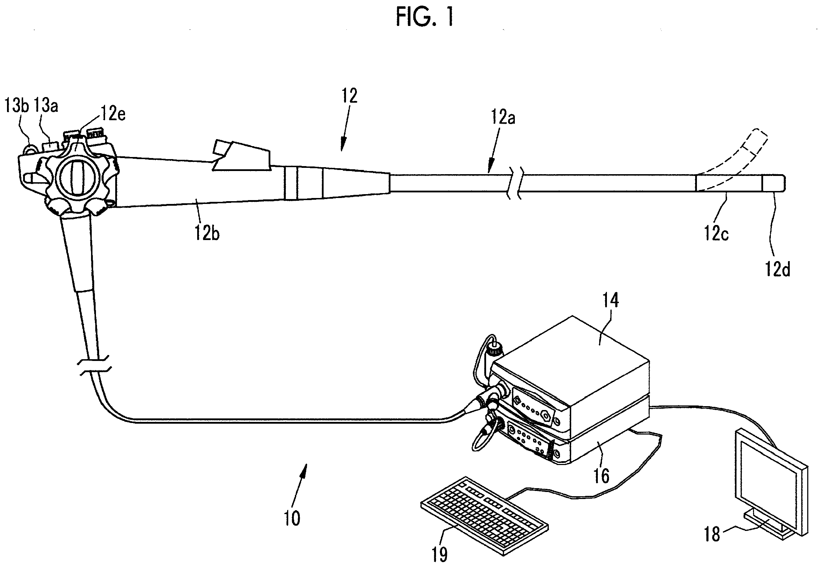

As illustrated in Fig. 1, an endoscope system 10 includes an endoscope 12, a light source device 14, a processor device 16, a monitor 18 (display unit), and a console 19. The endoscope 12 is optically connected to the light source device 14 and is electrically connected to the processor device 16. The endoscope 12 includes an insertion part 12a inserted into a subject, an operation unit 12b provided at a base end portion of the insertion part 12a, and a being portion 12c and a tip end portion 12d provided on a tip end side of the insertion part 12a. By operating an angle knob 12e of the operation unit 12b, the being portion 12c performs a bending operation. With this bending operation, the tip end portion 12d is directed in a desired direction.

Further, a mode changeover SW 13a and a zoom operation unit 13b are provided in the operation unit 12b, in addition to the angle knob 12e. The mode changeover SW 13a is used for a switching operation among four modes including a normal observation mode, a first special observation mode, a second special observation mode, and a simultaneous observation mode. The normal observation mode is a mode for displaying a normal image on the monitor 18. The first special observation mode is a mode for displaying the first special image in which the pit pattern on the observation target is clarified, on the monitor 18. The second special observation mode is a mode for displaying a second special image in which a mucous membrane in the first special image is represented with a normal color (color of a pink tone), on the monitor 18. The simultaneous observation mode is a mode for simultaneously displaying the first special image and the second special image on the monitor 18.

The processor device 16 is electrically connected to the monitor 18 and the console 19. The monitor 18 outputs and displays image information, or the like. The console 19 functions as a user interface (UI) that receives an input operation such as a function setting operation. An external recording unit (not illustrated) that records image information or the like may be connected to the processor device 16.

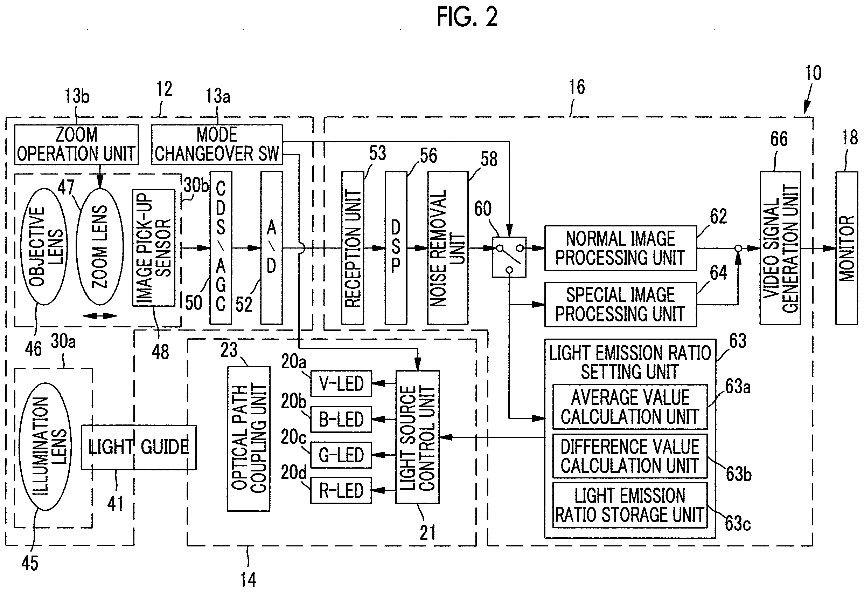

As illustrated in Fig. 2, the light source device 14 includes a violet light emitting diode (V-LED) 20a, a blue light emitting diode (B-LED) 20b, a green light emitting diode (G-LED) 20c, a red light emitting diode (R-LED) 20d, a light source control unit 21 that controls driving of the LEDs 20a to 20d of four colors, and an optical path coupling unit 23 that couple optical paths for light emitted from the LEDs 20a to 20d of four colors. The inside of the object is irradiated with light coupled in the optical path coupling unit 23, via a light guide 41 and an illumination lens 45 inserted into the insertion part 12a. A laser diode (LD) may be used in place of the LED. The "light source unit" of the present invention corresponds to a configuration including at least a B-LED 20b, a G-LED 20c, and an R-LED 20d.

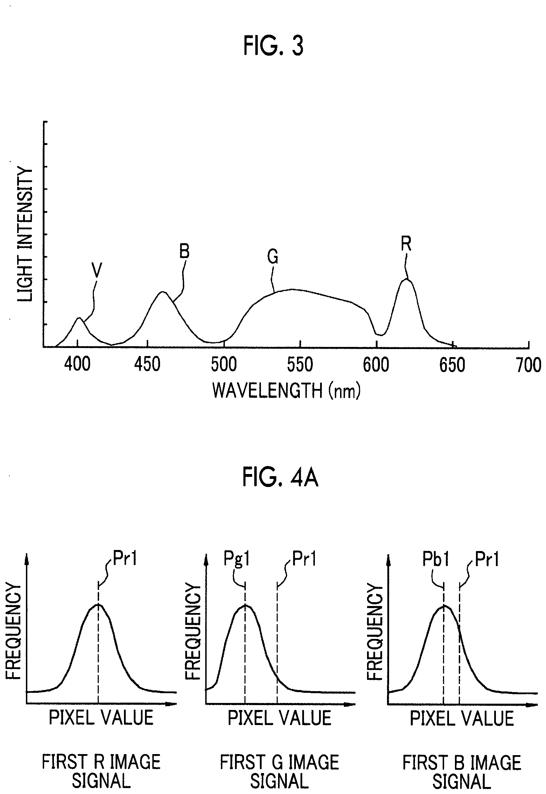

As illustrated in Fig. 3, the V-LED 20a generates a violet light V having a center wavelength of 405 nm and a wavelength range of 380 to 420 nm. The B-LED 20b generates a blue light B having a center wavelength of 460 nm and a wavelength range of 420 to 500 nm. The G-LED 20c generates a green light G having a wavelength range of 480 to 600 nm. The R-LED 20d generates a red light R having a center wavelength of 620 to 630 nm and a wavelength range of 600 to 650 nm. In the respective LEDs 20a to 20d, the center wavelength and a peak wavelength may be the same or may be different.

The light source control unit 21 may turn on all of the V-LED 20a, the B-LED 20b, the G-LED 20c, and the R-LED 20d in any one of the normal observation mode, the first special observation mode, the second special observation mode, and the simultaneous observation mode. In this case, the observation target is irradiated with light of four colors including the violet light V, the blue light B, the green light G, and the red light R. Light of at least three colors including the blue light B, the green light G, and the red light R may be emitted and may illuminate the observation target.

Further, the light source control unit 21 controls the respective LEDs 20a to 20d so that the light emission ratios of the violet light V, the blue light B, the green light G, and the red light R become a light emission ratio for normal observation in the normal observation mode. The light emission ratio for normal observation is not changed while the normal observation mode is being set. On the other hand, the light source control unit 21 controls the respective LEDs 20a to 20d so that the light emission ratios of the violet light V, the blue light B, the green light G, and the red light R become a light emission ratio for special observation in the first special observation mode, the second special observation mode, and the simultaneous observation mode. The light emission ratio for special observation is determined on the basis of an RGB image signal obtained by imaging the observation target.

As illustrated in Fig. 2, the light guide 41 is built into the endoscope 12 and a universal cord (a cord connecting the endoscope 12, and the light source device 14 and the processor device 16), and propagates light coupled by the optical path coupling unit 23 to the tip end portion 12d of the endoscope 12. A multi-mode fiber may be used as the light guide 41. For example, a small-diameter fiber cable having a core diameter of 105 µm, a cladding diameter of 125 µm, a diameter including a protective layer serving as an outer coat is φ 0.3 to 0.5 mm may be used.

An illumination optical system 30a and an image pick-up optical system 30b are provided in the tip end portion 12d of the endoscope 12. The illumination optical system 30a includes an illumination lens 45, and the observation target is irradiated the light from the light guide 41 via the illumination lens 45. The image pick-up optical system 30b includes an objective lens 46, a zoom lens 47, and an image pick-up sensor 48. Accordingly, reflected light from the observation target is incident on the image pick-up sensor 48 via the objective lens 46 and the zoom lens 47. Accordingly, a reflection image of the observation target is formed in the image pick-up sensor 48. Further, the zoom lens 47 is moved between a tele end and a wide end by operating the zoom operation unit 13b. In a case in which is enlargement observation is not performed (at the time of non-enlargement observation) , the zoom lens 47 is arranged at the wide end. In a case in which the enlargement observation is performed, the zoom lens 47 is moved from the wide end to the tele end through an operation of the zoom operation unit 13b.

The image pick-up sensor 48 is a color image pick-up sensor, and captures the reflection image of the object and outputs an image signal. It is preferable for this image pick-up sensor 48 to be a charge coupled device (CCD) image pick-up sensor or a complementary metal-oxide semiconductor (CMOS) image pick-up sensor, or the like. The image pick-up sensor 48 used in the present invention is a color image pick-up sensor for obtaining a RGB image signal of three colors of R (red), G (green), and B (blue), that is, a so-called RGB image pick-up sensor including an R pixel having an R filter provided therein, a B pixel having a B filter provided therein, and a G pixel having a G filter provided therein.

The image pick-up sensor 48 may be a so-called complementary color image pick-up sensor including complementary color filters of C (cyan), M (magenta) , Y (yellow), and G (green), rather than the image pick-up sensor of RGB colors. In a case in which the complementary color image pick-up sensor is used, an image signal of four colors including CMYG is output. Accordingly, it is necessary to convert an image signal of four colors including CMYG into an image signal of three colors including RGB through complementary color-primary color conversion. Further, the image pick-up sensor 48 may be a monochrome sensor in which no color filter is provided. In this case, it is necessary for the light source control unit 21 to cause the blue light B, the green light G, and the red light R to be emitted in time division, and to apply a synchronization process in processing of an imaging signal.

An image signal output from the image pick-up sensor 48 is transmitted to the CDS and AGC circuit 50. The CDS and AGC circuit 50 performs correlated double sampling (CDS) or automatic gain control (AGC) on the image signal that is an analog signal. The image signal through the CDS and AGC circuit 50 is converted into a digital image signal by the A/D converter 52. The digital image signal after A/D conversion is input to the processor device 16.

The processor device 16 includes a reception unit 53, a DSP 56, a noise removal unit 58, an image processing switching unit 60, a normal image processing unit 62, a light emission ratio setting unit 63, a special image processing unit 64, and a video signal generation unit 66. The reception unit 53 receives a digital RGB image signal from the endoscope 12. The R image signal (red image signal) corresponds to a signal that is output from the R pixel of the image pick-up sensor 48, the G image signal (green image signal) corresponds to a signal that is output from the G pixel of the image pick-up sensor 48, and the B image signal (blue image signal) corresponds to a signal that is output from the B pixel of the image pick-up sensor 48.

The DSP 56 performs gamma correction and color correction process on the RGB image signal. The noise removal unit 58 performs noise removal process (for example, a moving average method or a median filter method) on the RGB image signal subjected to the gamma correction and the like in the DSP 56 to remove noise from the RGB image signal. The RGB image signal from which the noise has been removed is transmitted to the image processing switching unit 60.

In a case in which the normal observation mode is set by the mode changeover SW 13a, the image processing switching unit 60 transmits the RGB image signal to the normal image processing unit 62. In a case in which the first special observation mode, the second special observation mode, or the simultaneous observation mode is set, the image processing switching unit 60 transmits the RGB image signal to the light emission ratio setting unit 63 and the special image processing unit 64.

The normal image processing unit 62 operates in a case in which the normal observation mode is performed, and performs a color conversion process, a color enhancement process, and a structure enhancement process on the RGB image signal. In the color conversion process, the normal image processing unit 62 performs 3x3 matrix processing, a gradation conversion process, three-dimensional LUT processing, and the like on the digital RGB image signal to convert the digital RGB image signal into the RGB image signal subjected to the color conversion process. Next, the normal image processing unit 62 performs various color enhancement processes on the RGB image signal subjected to the color conversion. The normal image processing unit 62 performs the structure enhancement process such as spatial frequency enhancement on the RGB image signal subjected to the color enhancement process. The RGB image signal subjected to the structure enhancement process is input as an RGB image signal of a normal image from the normal image processing unit 62 to the video signal generation unit 66.

The light emission ratio setting unit 63 sets the light emission ratio for special observation on the basis of the RGB image signal. Information on the set light emission ratio for a special observation is transmitted to the light source control unit 21. Details of the light emission ratio setting unit 63 will be described below.

The special image processing unit 64 operates in a case in which the first special observation mode, the second special observation mode, or the simultaneous observation mode is set, and generates the first special image, the second special image, and a simultaneous display special image for simultaneously displaying the first special image and the second special image. Details of this special image processing unit 64 will be described below. An RGB image signal of the first special image, the second special image,and the special image for a simultaneous display generated by the special image processing unit 64 is input to the video signal generation unit 66.

The video signal generation unit 66 converts the RGB image signal input from the normal image processing unit 62 or the special image processing unit 64 into a video signal for displaying the RGB image signal as an image that can be displayed on the monitor 18. On the basis of this video signal, the monitor 18 displays the normal image, the first special image, and the second special image or simultaneously displays the first special image and the second special image.

The light emission ratio setting unit 63 includes an average value calculation unit 63a, a difference value calculation unit 63b, and a light emission ratio storage unit 63c. The average value calculation unit 63a calculates an average value of the pixel values of the R image signal, an average value of the pixel values of the G image signal, and an average value of the pixel values of the B image signal on the basis of the RGB image signal. Next, the difference value calculation unit 63b subtracts the average value of the pixel values of the G image signal from the average value of the pixel values of the R image signal to calculate an RG difference value, and subtracts the average value of the pixel values of the B image signal from the average value of the pixel values of the R image signal to calculate an RB difference value.

The light emission ratio storage unit 63c stores a correspondence relationship between the RG difference and the RB difference value, and the light emission ratio of the violet light V, the blue light B, the green light G, and the red light R. In this correspondence relationship, a light emission amount of the green light G is set to increase as the RG difference value increases, and a light emission amount of the blue light G is set to increase as the RB difference value increases. The light emission ratio setting unit 63 sets the light emission ratio corresponding to the RG difference values and the RB difference value that have been calculated, as the light emission ratio for special observation, by referring to the correspondence relationship. The light source control unit 21 controls the respective LEDs 20a to 20d on the basis of the set light emission ratio for special observation, and controls the light amount of the violet light V, the blue light B, the green light G, and the red light R. A "control unit" of the present invention corresponds to a configuration including the "light emission ratio setting unit 63" and the "light source control unit 21".

Hereinafter, a method of setting the light emission ratio in the light emission ratio setting unit 63 will be described. First, assuming that switching to the first special observation mode is performed by the mode changeover SW 13a, the light source control unit 21 performs control so that light violet light V, the blue light B, the green light G, and the red light R are emitted at the first light emission ratio. In this case, in the first RGB image signal obtained on the basis of the first light emission ratio, the most frequent value Pg1 of the first G image signal and the most frequent value Pb1 of the first B image signal are both smaller than the most frequent value Pr1 of the first R image signal, as illustrated in FIG 4A. That is, the signal level of the first R image signal is higher than the signal level of the first G image signal or the first B image signal.

This is significantly affected by the fact that hemoglobin is contained in the observation target. Further, in a case in which the pyoktanin coloring agent is sprayed to the observation target, two light absorption substances including hemoglobin and pyoktanin coloring agent are contained in in the observation target. Since this pyoktanin coloring agent has a green light G absorption characteristic, a most frequent value Pg1 of the first G image signal in the first RGB image signal is particularly small due to mixing of the pyoktanin coloring agent. In this case, the pit pattern is clarified due to spraying of the pyoktanin coloring agent on the first special image obtained on the basis of the first RGB image signal, but the brightness of the pit pattern is slightly darkened with a decrease in the pixel value of the first G image signal.

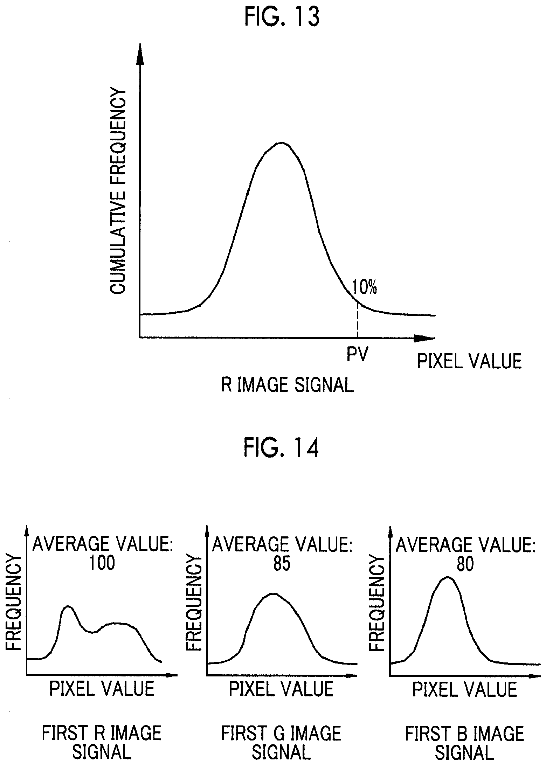

The light emission ratio setting unit 63 sets the light emission ratio for special observation to the first light emission ratio until receiving a light emission ratio change instruction according to an instruction from the console 19 or the like. It is preferable for the first light emission ratio to be set to the same light emission ratio as the light emission ratio for normal observation. In a case in which the first light emission ratio is set to the same light emission ratio as the light emission ratio for normal observation, the light amount control of the violet light V, the blue light B, the green light G, and the red light R is performed, as follows. First, a luminance signal generation unit (not illustrated) in the processor device 16 creates a Y (luminance) signal on the basis of the first RGB image signal and transmits the Y signal to the light source control unit 21. Then, the light source control unit 21 controls the respective LEDs 20a to 20d while maintaining a constant ratio on the basis of a histogram of the Y signal to control the intensity of the violet light V, the blue light B, the green light G, and the red light R. A method of adjusting the intensity so that an average value or a 10% peak value (corresponding to "PV" in a case in which a horizontal axis is "luminance" in Fig. 13) in the histogram of the Y signal matches a predetermined criterion can be considered as the control on the basis of histogram of the Y signal.

Next, assuming that the light emission ratio setting unit 63 receives the light emission ratio change instruction, the average value calculation unit 63a calculates the average value of the pixel values of the first RGB image signal on the basis of the first RGB image signal, and the difference value calculation unit 63b calculates a RG difference and a RB difference value on the basis of the calculated pixel value of the first RGB image signal. The light emission ratio setting unit 63 sets the second light emission ratio as the light emission ratio for special observation on the basis of the RG difference value and the RB difference value that have been calculated.

The light source control unit 21 performs changing from the first light emission ratio to the second light emission ratio, and performs emission of the violet light V, the blue light B, the green light G, and the red light R at the second light emission ratio. In this case, the light source control unit 21 controls the light amount of the violet light V, the blue light B, the green light G, and the red light R so that the average values of the pixel values of the first G image signal and the first B image signal match the average value of the pixel values of the first R image signal.

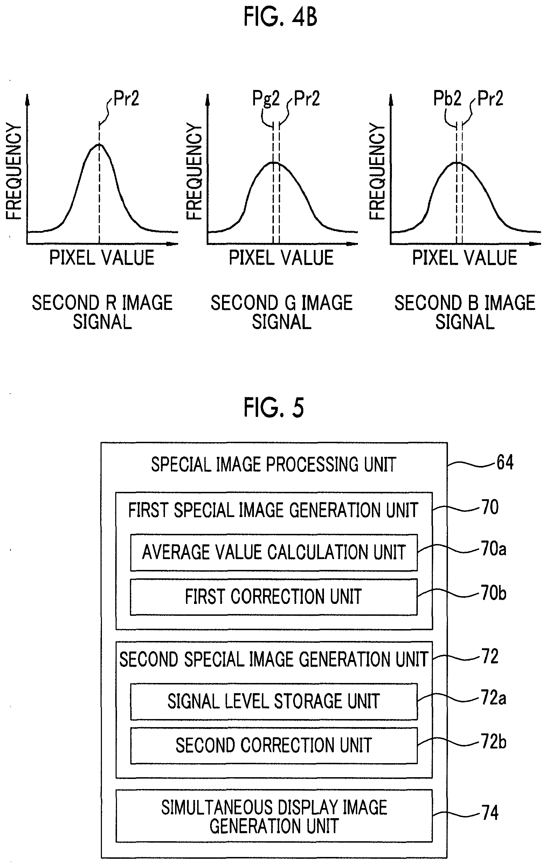

In the second RGB image signal obtained on the basis of the second light emission ratio, the most frequent value Pg2 of the second G image signal and the most frequent value Pb2 of the second B image signal approach the most frequent value Pr2 of the second R image signal, as illustrated in Fig. 4B. A width of the histogram of the second G image signal and the second B image signal has been widened. This is because the light amounts of the green light G and the blue light B have increased through the light amount control of the light source control unit 21 based on the second light emission ratio. Further, by increasing the amount of the green light G, it is possible to increase the pixel value of the second image signal, in other words, increase the brightness. Accordingly, it is possible to improve the brightness of the pit pattern even in the event that the pyoktanin coloring agent is sprayed.

As illustrated in Fig. 5, the special image processing unit 64 includes a first special image generation unit 70, a second special image generation unit 72, and a simultaneous display image generation unit 74. The first special image generation unit 70 generates the first special image on the basis of the second RGB image signal obtained in the event that the violet light V, the blue light B, the green light G, the red light R are emitted at the second light emission ratio.

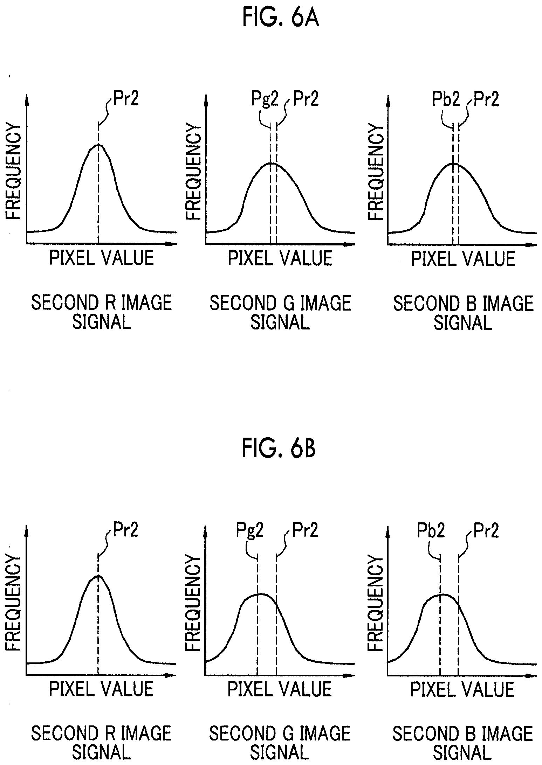

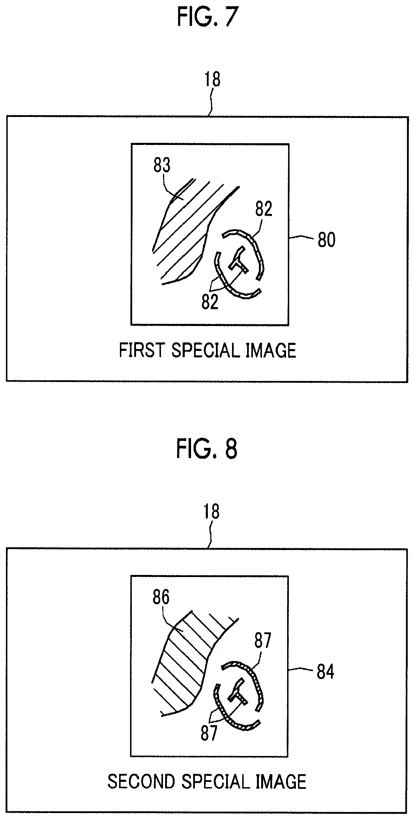

The first special image generation unit 70 includes an average value calculation unit 70a, and a first correction unit 70b. The average value calculation unit 70a calculates the average value of the pixel values of the second R image signal, the average value of the pixel values of the second G image signal, and the average value of the pixel values of the second B image signal on the basis of the second RGB image signal. In a case in which the average value of the pixel values of the second G image signal and the second B image signal is within a specific range, the most frequent value Pg2 of the second G image signal and the most frequent value Pb2 of the second B image signal approach the most frequent value Pr2 of the second R image signal, as illustrated in Fig. 6A. In this case, the first special image generation unit 70 generates the first special image on the basis of the second RGB image signal. As illustrated in Fig. 7, the brightness of the pit pattern 82 on the first special image 80 is sufficiently improved. It is preferable for the specific range to be a range between a value obtained by subtracting a certain pixel value (appropriately set by the user) from the average value of the pixel values of the second R image signal and a value obtained by adding a certain pixel value (appropriately set by the user) to the average value of the pixel values of the second R image signal.

On the other hand, in a case in which the average value of the pixel values of the second G image signal and the second B image signal is outside a specific range, the most frequent value Pg2 of the second G image signal and the most frequent value Pb2 of the second B image signal is smaller than the most frequent value Pr2 of the second R image signal, as illustrated in Fig. 6B. It is considered that this is because the pixel value of the second G image signal or the pixel value of the second B image signal can be sufficiently increased through light amount control by the light source control unit 21.

In this case, the first correction unit 70b corrects the second G image signal or the second B image signal so that the average value of the pixel values of the second G image signal and the average value of the pixel values of the second B image signal match the average value of the pixel values of the second R image signal. In the second RGB image signal after correction, the most frequent value Pg2 of the second G image signal and the most frequent value Pb2 of the second B image signals are substantially the same as the most frequent value Pr2 of the second R image signal. The first special image generation unit 70 generates the first special image on the basis of the second R image signal, the second G image signal after correction, and the second B image signal after correction. Accordingly, brightness of the pit pattern is sufficiently improved on the first special image 80.

A method of calculating a difference value between the average value of the pixel values of the second G image signal and the average value of the pixel values of the second R image signal (or a difference value between the average value of the pixel values of the second B image signal and the average value of the pixel values of the second R image signal) and correcting the second G image signal (or the second B image signal) so that the difference value decreases can be considered as the correction method in the first correction unit 70b.

The second special image generation unit 72 illustrated in Fig. 5 generates a second special image on the basis of the second RGB image signal obtained in the event that the violet light V, the blue light B, the green light G, and the red light R are emitted at the second light emission ratio. Here, since the first special image generated by the first special image generation unit 70 increases the pixel values of the second G image signal and the second B image signal, a mucous membrane 83 on the first special image 80 is displayed with a color such as green or blue (see Fig. 7) other than the normal color, rather than a normal color (pink) . Thus, in a case in which the color of the mucous membrane is displayed with the color other than the normal color, a doctor cannot reliably perform diagnosis in some cases. Therefore, the second special image generation unit 72 generates the second special image obtained by returning the color of the mucous membrane of the first special image to the normal color.

The second special image generation unit 72 includes a signal level storage unit 72a, and a second correction unit 72b. The signal level storage unit 72a calculates the average value of the pixel values of the first RGB image signal on the basis of the first RGB image signal obtained in the event that the violet light V, the blue light B, the green light G, and the red light R are emitted at the first light emission ratio, and stores the calculated average value of the pixel values of the first RGB image signal.

The second correction unit 72b corrects the second RGB image signal on the basis of the average value of the pixel values of the first RGB image signal stored in the signal level storage unit 72a. The second correction unit 72b corrects the second G image signal so that the average value of the pixel values of the second G image signal becomes the average value of the pixel values of the first G image signal, or corrects the second B image signal so that the average value of the pixel values of the second B image signal becomes the average value of the pixel values of the first B image signal. Since the average value of the pixel values of the second G image signal is greater than the average value of the pixel values of the first G image signal, the average value of the pixel values of the second G image signal decreases through correction (in addition, a width of a histogram decreases). Similarly, the average value of the pixel values of the second B image signal decreases through correction.

The second special image generation unit 72 generates a second special image on the basis of the second R image signal, the second G image signal after correction, and the second B image signal after correction. As illustrated in Fig. 8, the mucous membrane 86 on the second special image 84 is displayed in a color of a pink tone that is a normal color. However, on the second special image 84, brightness of a pit pattern 87 is darkened upon being compared to the first special image 80.

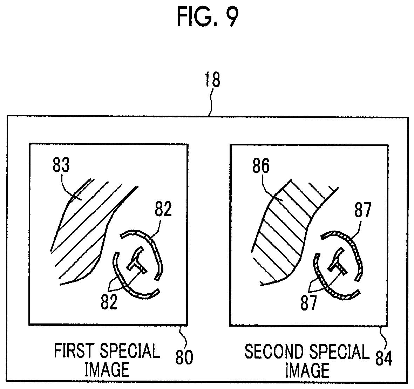

The simultaneous display image generation unit 74 generates a simultaneous display special image on the basis of the first special image and the second special image generated by the first special image generation unit 70 and the second special image generation unit 72. The monitor 18 displays the first special image 80 on one side and displays the second special image 84 on the other side on the basis of the simultaneous display special image, as illustrated in Fig. 9. By simultaneously displaying the first special image 80 and the second special image 84 in this way, it is possible to recognize the mucous membrane 86 from the second special image 84 with the normal color (pink color tone) while recognizing the pit pattern 82 with improved brightness from the first special image 80.

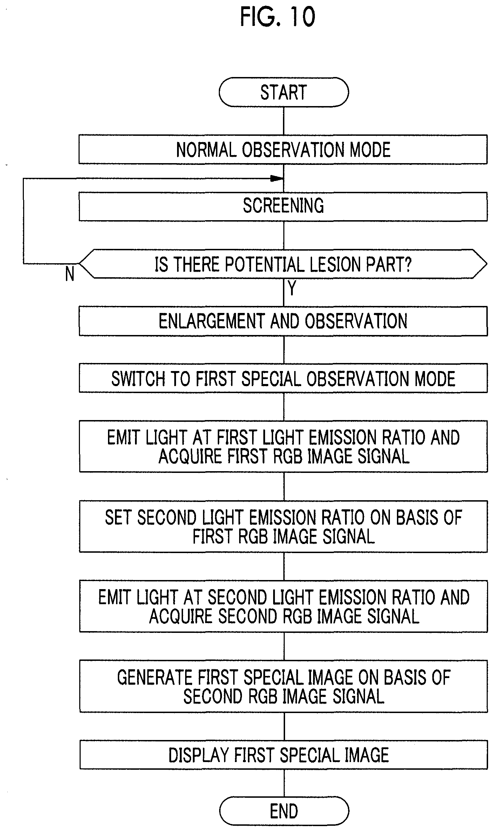

Next, a series of flow in this embodiment will be described according to a flowchart of Fig. 10. First, in the normal observation mode, screening is performed from a distant view state. At the time of this screening, in the event that a part (potential lesion part) that is likely to be a lesion such as a Brownish area or reddening is detected, the zoom operation unit 13b is operated and enlargement and observation is performed to enlarge and display the observation target including the potential lesion part. Accordingly, the mode changeover SW 13a is operated for switching to the first special observation mode, and a pyoktanin coloring agent is sprayed to be observation target.

First, in the first special observation mode, the violet light V, the blue light B, the green light G, and the red light R are emitted at a first light emission ratio. The observation target to which the pyoktanin coloring agent has been sprayed is irradiated with the light of the four colors emitted at the first light emission ratio. This observation target is imaged by the image pick-up sensor 48 and the first RGB image signal is obtained. Light emission is performed at the first light emission ratio until the light emission ratio setting unit 63 receives the light emission ratio change instruction. Assuming that the light emission ratio setting unit 63 receives the light emission ratio change instruction, the light emission ratio setting unit 63 newly sets the light emission ratio of the violet light V, the blue light B, the green light G, and the red light R as the second light emission ratio on the basis of the first RGB image signal. Information on the newly set second light emission ratio is sent to the light source control unit 21.

The light source control unit 21 controls the respective LEDs 20a to 20d on the basis of the second light emission ratio and adjusts the light amount of the violet light V, the blue light B, the green light G, and the red light R. In the light source control unit 21, the amount of the blue light B and the green light G is adjusted so that the average values of the pixel values of the first G image signal and the first B image signal match the average value of the pixel values of the first R image signal. For example, the pixel value of the first G image signal decreases in the event that pyoktanin is sprayed, but the light source control unit 21 increases at least the amount of the green light G so as to compensate for the decrease in the pixel value of the first G image signal. Next, the first special image is generated from the second RGB image signal that is obtained on the basis of the second light emission ratio. The first generated special image is displayed on the monitor 18. On the first special image, the brightness of the pit pattern is improved.

In a case in which the mucous membrane is displayed with the normal color (pink tone), the mode changeover SW 13a is operated, switching to the second special observation mode is performed, and the second special image is displayed on the monitor 18. Further, in a case in which the mucous membrane of the normal color is displayed while displaying the pit pattern in a bright state, the mode changeover SW 13a is operated and switching to the simultaneous observation mode is performed. By this switching operation, the first special image and the second special image are simultaneously displayed on the monitor 18.

In the above embodiment, after the second light emission ratio is set by the light emission ratio setting unit 63, the light emission ratio for special observation may be fixed to the second light emission ratio and the emission of the violet light V, the blue light B, the green light G, and the red light R may be performed. Further, the second light emission ratio may be changed each time the observation target is imaged by the image pick-up sensor 48, and the emission of the violet light V, the blue light B, the green light G, and the red light R may be performed on the basis of the second light emission ratio after changing.

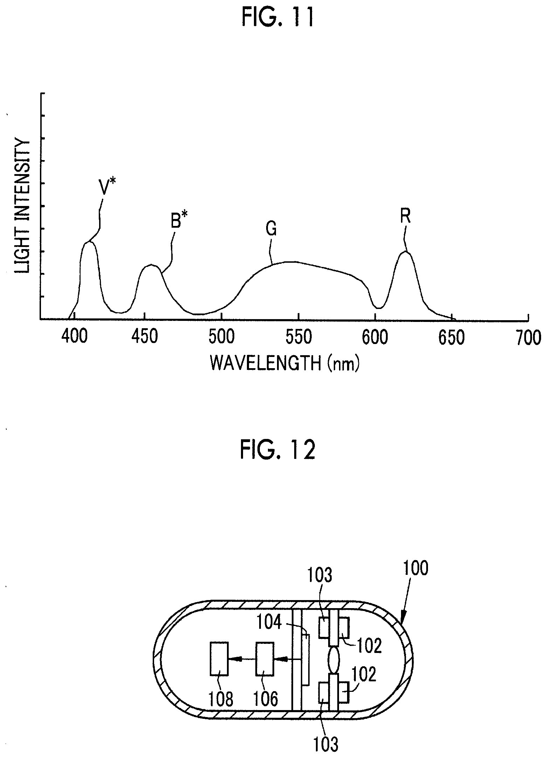

In the above embodiment, the light of four colors having the light emission spectrum as illustrated in Fig. 3 has been used, but the light emission spectrum is not limited thereto. For example, as illustrated in Fig. 11, the green light G and the red light R have the same spectrum as that in Fig. 3, whereas the violet light V* may be light having a center wavelength of 410 to 420 nm and a wavelength range on slightly longer wavelength side than the violet light V in Fig. 3. The blue light B* may be light having a center wavelength of 445 to 460 nm and a wavelength range on a slightly shorter wavelength side than the blue light B in Fig. 3 .

Although the present invention is embodied in the endoscope system in which the endoscope 12 having the image pick-up sensor 48 provided therein is inserted into a body and observation is performed in the above embodiment, the present invention may be embodied in a capsule endoscope system. For example, the capsule endoscope system includes at least a capsule endoscope 100 illustrated in Fig. 12, and a processor device (not illustrated) .

The capsule endoscope 100 includes an LED 102, a light source control unit 103, an image pick-up sensor 104, an image processing unit 106, and a transmission and reception antenna 108 . The LED 102 includes a V-LED that emits violet light V, a B-LED that emits blue light B, a G-LED that emits green light G, and a R-LED that emits red light R. The violet light V, the blue light B, the green light G, and the red light R are emitted from the LEDs of the four colors. The violet light V, the blue light B, the green light G, and the red light R emitted from the LEDs 102 illuminate the observation target. A laser diode (LD) may be used in place of the LED.

The light source control unit 103 controls driving of the LED 102. This light source control unit 103 can wirelessly communicate with the processor device of the capsule endoscope system via the transmission and reception antenna 108. The processor device of the capsule endoscope system is substantially the same as the processor device 16 shown in the above embodiment. The processor device of the capsule endoscope system transmits information on the light emission ratio set by the light emission ratio setting unit 63 to the capsule endoscope 100. The light source control unit 103 controls the driving of the LED 102 on the basis of the received information on the light emission ratio.

The image pick-up sensor 104 is a color image pick-up sensor, and images an observation target illuminated by white light and outputs an RGB image signal. Here, it is preferable for a charge coupled device (CCD) image pick-up sensor or a complementary metal-oxide semiconductor (CMOS) image pick-up sensor to be used as the image pick-up sensor 104. The RGB image signal output from the image pick-up sensor 104 is subjected to a process for conversion to a signal that can be transmitted using the transmission and reception antenna 108 in the image processing unit 106. The RGB image signal through the image processing unit 106 is wirelessly transmitted from the transmission and reception antenna 108 to a processor device of a capsule endoscope system.

In the above embodiment, a most frequent value in a histogram of the RGB image signal, a 10% peak value indicating a pixel value at which a cumulative frequency is 10% upon being viewed from a high pixel value side (for example, a pixel value PV in the case of Fig. 13) in the histogram of the RGB image signal, or the like may be used as a value indicating the signal level of the RGB image signal, in addition to the average value of the pixel values of the RGB image signal.

Although the intensity of the violet light V, the blue light B, the green light G, and the red light R is adjusted so that the signal level of the first G image signal or the first B image signal matches the signal level of the first R image signal in order to improve brightness of the pit pattern in the event that the pyoktanin coloring agent is sprayed in the above embodiment, the intensity of the light of each color may be adjusted so that the signal level of the first R image signal or the first B image signal matches the first G image signal according to various purposes, or the intensity of the light of each color may be adjusted so that the signal level of the first R image signal or the first G image signal matches the signal level of the first B image signal. That is, the intensity of the light of each color is adjusted so that the signal levels of the image signal of the second color or the image signal of the third color match the signal level of the image signal of the first color according to various purposes .

For example, in the event that the pyoktanin coloring agent is sprayed, the brightness of the pit pattern is improved, and in a case in which some colors are left in the event that the pyoktanin coloring agent is sprayed, it is preferable to adjust the intensity of the violet light V, the blue light B, the green light G, and the red light R so that the target value of the R image signal x α = the target value of the G image signal, and the target value of the R image signal x β = the target value of the B image signal. In this case, it is preferable to newly provide a pyoktanin coloring agenting enhancement mode as the observation mode in order to perform the intensity adjustment as described above.

Further, although the intensity of the violet light V, the blue light B, the green light G, and the red light R has been adjusted in the event that the pyoktanin coloring agent is sprayed in the above embodiment, the intensity of the light of each color may be adjusted so that levels of the image signal of the second color or the image signal of the third color match the signal level of the image signal of the first color in the event that iodine (which absorbs blue in illumination light, slightly absorbs green, and emits orange) or indigo carmine (which absorbs red and green in the illumination light and emits blue) is sprayed.

For example, in a case in which the indigo carmine is sprayed, the first RGB image signal having a histogram as illustrated in Fig. 14 is obtained in the event that the violet light V, the blue light B, the green light G, and the red light R are emitted at the first light emission ratio. The first R image signal is substantially evenly distributed from a high pixel value to a low pixel value. The average value of the pixel values of the first R image signal is approximately "100" and is somewhat smaller than the average value of the R image signal in a case in which another coloring agent such as iodine or pyoktanin is used. This is because the indigo carmine absorbs a red component of illumination light. On the other hand, the first G image signal and the first B image signal are distributed from an intermediate pixel value to a low pixel value. The average value of the pixel values of the first G image signal is approximately "85", and the average value of the pixel values of the first B image signal is approximately "80".

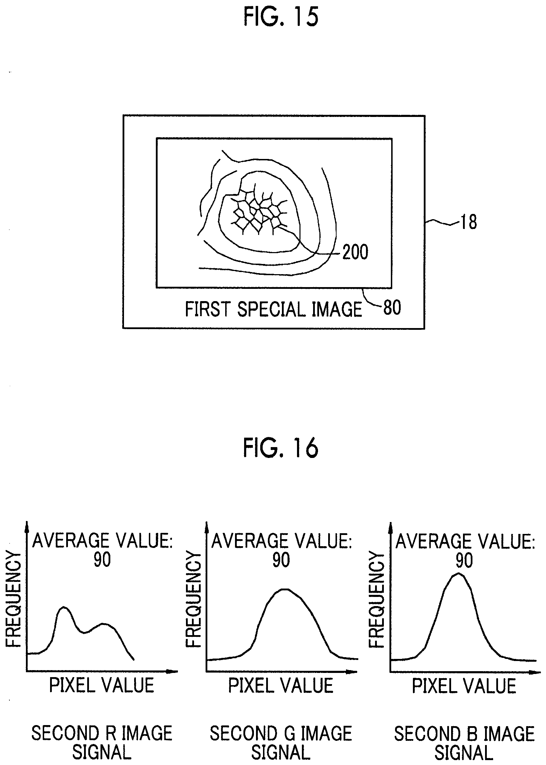

As described above, it is preferable that the light emission ratio of the violet light V, the blue light B, the green light G, and the red light R is changed from the first light emission ratio to the second light emission ratio and the light amount control is performed even in a case in which the indigo carmine is sprayed so that the average values of the first G image signal and the first B image signal match the average value of the first R image signal, as in a case in which the pyoktanin is sprayed. The method of setting the light emission ratio or the light amount control based on the second light emission ratio is the same as those in the above embodiment. In the second RGB image signal obtained by the light amount control based on the second light emission ratio, the pixel values of the second G image signal and the second B image signal increase such that contrast of the pit pattern 200 into which the indigo carmine flows is improved, as illustrated in Fig. 15.

Further, the first target value may be determined from the first R image signal, instead of setting the second light emission ratio, so that the average values of the first G image signal and the first B image signal match the average value of the first R image signal, and the third light emission ratio may be set so that the average values of the first R image signal, the first G image signal, and the first B image signal match the first target value. It is preferable for the first target value to be set to a value slightly smaller than the average value of the first R image signal. The setting of the first target value is performed by a target value setting unit (not illustrated) provided inside the light emission ratio setting unit 63.

For example, in a case in which the average value of the first R image signal is approximately "100", the first target value is set to approximately "90" , and third light emission ratio is set so that the average value of the first R image signal, the first G image signal, and the first B image signal approaches approximately the first target value "90" . The second RGB image signal having a histogram as illustrated in Fig. 16 can be obtained by performing the light amount control of the violet light V, the blue light B, the green light G, and the red light R on the basis of the third set light emission ratio. In the second RGB image signal, the pixel value of the second G image signal and the second B image signal is greater than that of the first RGB image signal, as described above. On the other hand, since the average value of the second R image signal is smaller than that of the first RGB image signal, a pixel distribution of a high pixel value is low and a distribution range is narrow. Accordingly, it is possible to further improve the contrast of the pit pattern 200 into which the indigo carmine flows, as compared with the case in which the light amount control is performed on the basis of the second light emission ratio.

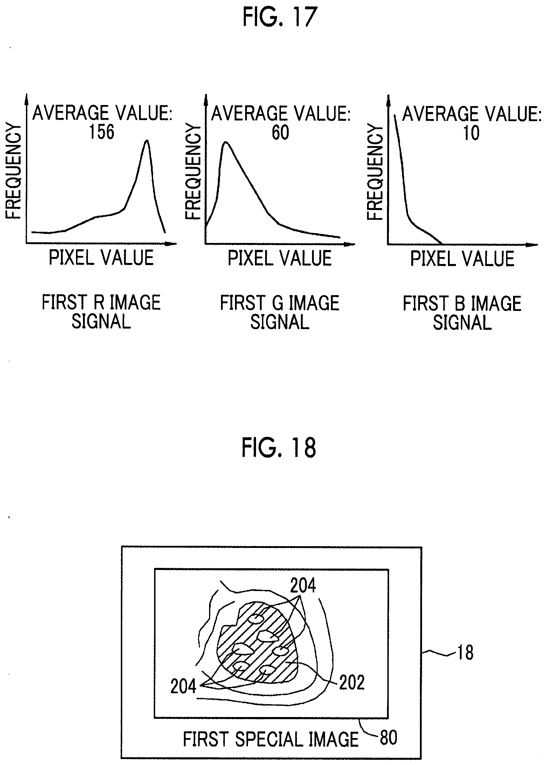

Further, in a case in which iodine is sprayed, in the event that the violet light V, the blue light B, the green light G, and the red light R are emitted at the first light emission ratio, the first RGB image signal having a histogram as illustrated in Fig. 17 can be obtained. The first R image signal is distributed from a high pixel value to an intermediate pixel value, and a frequency of a part with the high pixel value is particularly high. The average value of the pixel values of the first R image signal is approximately "156". On the other hand, the first G image signal is distributed from an intermediate pixel value to a low pixel value, and a frequency of a part of the low pixel value is high. An average value of the pixel values of the first G image signal is approximately "60" . Further, the first B image signal is distributed from the intermediate pixel value to a low pixel value, similar to the first G image signal, but the frequency of a part with the low pixel value is very high. This is because absorption of the iodine for a blue component of illumination light is great. The average value of the pixel values of the first B image signal is approximately "10".

As described above, it is preferable that the light emission ratio of the violet light V, the blue light B, the green light G, and the red light R is changed from the first light emission ratio to the second light emission ratio and the light amount control is performed even in a case in which the indigo carmine is sprayed so that the average values of the first G image signal and the first B image signal match the average value of the first R image signal, as in a case in which the pyoktanin is sprayed. The method of setting the light emission ratio or the light amount control based on the second light emission ratio is the same as those in the above embodiment. In the second RGB image signal obtained by the light amount control based on the second light emission ratio, the pixel values of the second G image signal and the second B image signal increase such that a difference between an iodine stained area 202 in which iodine has been stained and an iodine non-strained area 204 in which the iodine has not been stained is clarified, as illustrated in Fig. 18.

Further, the first target value may be determined from the first R image signal, the second target value may be determined from the first G image signal, and the third target value may be determined from the first B image signal, instead of setting the second light emission ratio, so that the average values of the first G image signal and the first B image signal match the average value of the first R image signal, and then, the fourth light emission ratio may be set so that the average value of the first R image signal matches the first target value, the average value of the first G image signal matches the second target value, and the average value of the first B image signal matches the third target value. The setting of the first to third target values is performed by a target value setting unit (not illustrated) provided inside the light emission ratio setting unit 63.

It is preferable for the first target value to be set to a value slightly smaller than the average value of the first R image signal. On the other hand, it is preferable for the second target value to be set to a value greater than the average value of the first G image signal and smaller than the first target value. further, it is preferable for the third target value to be set to a value greater than the average value of the first B image signal and smaller than the first target value. However, assuming that the third target value is set to be too great, it is difficult for a difference between the iodine stained area 202 and the iodine non-strained area 204 to be recognized. Accordingly, it is preferable that a value obtained by adding a value in a range from "10" to "20" to the average value of the first B image signal is set as the third target value.

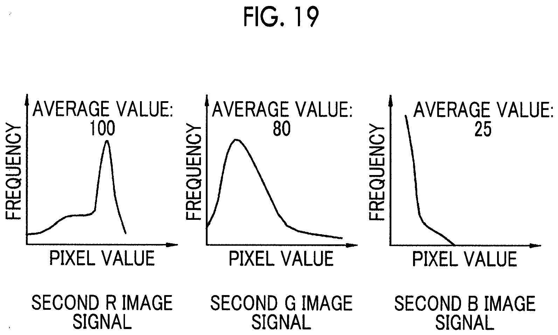

For example, the first target value is set to approximately "100" in a case in which the average value of the first R image signal is approximately "155", the second target value is set to approximately "80" in a case in which the average value of the first G image signal is approximately "60", and the third target value is set to approximately "25" in a case in which the average value of the first B image signal is approximately "10". The fourth light emission ratio is set so that the average value of the first R image signal matches the first target value, the average value of the first G image signal matches the second target value, and the average value of the first B image signal matches the third target value. The second RGB image signal having a histogram as illustrated in Fig. 19 can be obtained by performing the light amount control of the violet light V, the blue light B, the green light G, and the red light R on the basis of the fourth set light emission ratio.

In the second RGB image signal, the pixel values of the second G image signal and the second B image signal are greater than the pixel values of the first G image signal and the first B image signal, and a distribution range of the pixel values thereof is wider than that of the first G image signal and the first B image signal. On the other hand, in the second R image signal, the pixel distribution of the high pixel value disappears, the distribution range is narrow, and the most frequent value is shifted from a portion of the high pixel value to a portion of an intermediate pixel value. Accordingly, it is possible to further clarify a difference between the iodine stained area 202 in which iodine has been stained and the iodine non-strained area 204 in which the iodine has not been stained, as compared with a case in which the light amount control is performed on the basis of the second light emission ratio.

### [Additional Item]

An endoscope system comprising:
a light source unit that emits red light, green light, and blue light,
an image pick-up sensor that images an observation target in a body illuminated by the red light, the green light, and the blue light and outputs an image signal of a first color, an image signal of a second color, and an image signal of a third color,
a light emission ratio setting unit that sets a light emission ratio for causing a signal level of image signal of the second color or the image signal of the third color to match a signal level of the image signal of the first color, and
a light source control unit that controls the amount of light of at least one color among the red light, the green light, and the blue light on the basis of the light emission ratio set by the light emission ratio setting unit.

### Explanation of References

- 10:: endoscope system
- 18:: monitor (display unit)
- 20b:: B-LED
- 20c:: G-LED
- 20d:: R-LED
- 21:: light source control unit
- 48,104:: image pick-up sensor
- 63a:: average value calculation unit
- 70b:: first correction unit
- 72a:: signal level storage unit
- 72b:: second correction unit

## Claims

1. An endoscope system (10) comprising:
a light source unit (14) that emits red light, green light, and blue light at a first light emission ratio;
an image pick-up sensor (48, 104) that images an observation target in a body, and outputs a color image signal including an image signal of a first color, an image signal of a second color, and an image signal of a third color, the image pick-up sensor (48, 104) imaging the observation target in the body illuminated by the red light, the green light, and the blue light at the first light emission ratio and outputting a first color image signal; and
a control unit (21, 63) configured to change a light emission ratio of the red light, the green light, and the blue light of the light source unit (14) from the first light emission ratio to a second light emission ratio and control the amount of light of at least one color among the red light, the green light, and the blue light so that a signal level of the image signal of the second color or the image signal of the third color in the first color image signal matches a signal level of the image signal of the first color, and
wherein the image pick-up sensor (48, 104) images the observation target illuminated by the red light, the green light, and the blue light at the second light emission ratio, and outputs a second color image signal.

2. The endoscope system (10) according to claim 1, wherein the image signal of the first color is a red image signal, the image signal of the second color is a green image signal, and the image signal of the third color is a blue image signal,
the first color image signal is a first red image signal, a first green image signal, or a first blue image signal, and
the second color image signal is a second red image signal, a second green image signal, or a second blue image signal.

3. The endoscope system (10) according to claim 2, further comprising:
a first correction unit (70b) that corrects the second green image signal or the second blue image signal so that the signal level of the second green image signal or the second blue image signal matches a signal level of the second red image signal in a case in which the signal level of the second green image signal or the second blue image signal is outside a specific range.

4. The endoscope system (10) according to claim 2, further comprising:
a signal level storage unit (72a) that stores a signal level of the first green image signal or the first blue image signal; and
a second correction unit (72b) that corrects the second green image signal so that the signal level of the second green image signal becomes a signal level of the first green image signal, or corrects the second blue image signal so that a signal level of the second blue image signal becomes a signal level of the first blue image signal.

5. The endoscope system (10) according to claim 4, further comprising:
a display unit (18) that displays a first special image on the basis of the second red image signal, the second green image signal, and the second blue image signal, and displays a second special image on the basis of the second red image signal, the second green image signal after correction, and the second blue image signal after correction.

6. The endoscope system (10) according to any one of claims 2 to 5, further comprising:
an average value calculation unit (63a) that calculates an average value of the pixel values of the first red image signal, an average value of the pixel values of the first green image signal, and an average value of the pixel values of the first blue image signal on the basis of the first red image signal, the first green image signal, and the first blue image signal,
wherein the control unit (21) controls the amount of light of at least one color among the red light, the green light, and the blue light so that the average value of the pixel values of the first green image signal or the first blue image signal matches the average value of the pixel values of the first red image signal.

7. The endoscope system (10) according to any one of claims 1 or 2, further comprising:
a target value setting unit that sets a first target value from the signal level of the image signal of the first color in the first color image signal,
wherein the control unit (21) changes the light emission ratio of the red light, the green light, and the blue light from the first light emission ratio to a third light emission ratio, instead of changing the light emission ratio of the red light, the green light, and the blue light from the first light emission ratio to the second light emission ratio, and controls the amount of light of at least one color among the red light, the green light, and the blue light so that respective signal levels of the image signal of the first color, the image signal of the second color, and the image signal of the third color in the first color image signal match the first target value.

8. The endoscope system (10) according to any one of claims 1 or 2, further comprising:
a target value setting unit that sets a first target value from the signal level of the image signal of the first color in the first color image signal, sets a second target value from the signal level of the image signal of the second color in the first color image signal, and sets a third target value from the signal level of the image signal of the third color in the first color image signal,
wherein the control unit (21) changes the light emission ratio of the red light, the green light, and the blue light from the first light emission ratio to a fourth light emission ratio, instead of changing the light emission ratio of the red light, the green light, and the blue light from the first light emission ratio to the second light emission ratio, and controls the amount of light of at least one color among the red light, the green light, and the blue light so that the signal level of the image signal of the first color in the first color image signal matches the first target value, the signal level of the image signal of the second color in the first color image signal matches the second target value, and the signal level of the image signal of the third color in the first color image signal matches the third target value.

## Patentansprüche

1. Endoskopisches System (10) umfassend:
eine Lichtquelleneinheit (14), die rotes Licht, grünes Licht und blaues Licht in einem ersten Lichtemissionsverhältnis emittiert;
einen Bildaufnahmesensor (48, 104), der ein Betrachtungsziel in einem Körper abbildet und ein Farbbildsignal ausgibt, welches ein Bildsignal einer ersten Farbe, ein Bildsignal einer zweiten Farbe und ein Bildsignal einer dritten Farbe enthält, wobei der Bildaufnahmesensor (48, 104) das Betrachtungsziel in dem Körper abbildet, welches von dem roten Licht, dem grünen Licht und dem blauen Licht mit dem ersten Lichtemissionsverhältnis beleuchtet wird, und ein erstes Farbbildsignal ausgibt; und
eine Steuereinheit (21, 63), konfiguriert zum Ändern eines Lichtverhältnisses des roten Lichts, des grünen Lichts und des blauen Lichts der Lichtquelleneinheit (14) von dem ersten Lichtemissionsverhältnis zu einem zweiten Lichtemissionsverhältnis, und zum Steuern der Lichtmenge mindestens einer Farbe von dem roten Licht, dem grünen Licht und dem blauen Licht, so dass ein Signalpegel des Bildsignals der zweiten Farbe oder des Bildsignals der dritten Farbe innerhalb des ersten Farbbildsignals übereinstimmt mit einem Signalpegel des Bildsignals der ersten Farbe, und
wobei der Bildaufnahmesensor (48, 104) das Betrachtungsziel abbildet, welches von dem roten Licht, dem grünen Licht und dem blauen Licht in dem zweiten Lichtemissionsverhältnis beleuchtet wird, und ein zweites Farbbildsignal ausgibt.

2. Endoskopsystem (10) nach Anspruch 1, bei dem das Bildsignal der ersten Farbe ein rotes Bildsignal ist, das Bildsignal der zweiten Farbe ein grünes Bildsignal ist und das Bildsignal der dritten Farbe ein blaues Bildsignal ist,
das erste Farbbildsignal ein erstes rotes Bildsignal, ein erstes grünes Bildsignal oder ein erstes blaues Bildsignal ist, und
das zweite Farbbildsignal ein zweites rotes Bildsignal, ein zweites grünes Bildsignal oder ein zweites blaues Bildsignal ist.

3. Endoskopsystem (10) nach Anspruch 2, weiterhin umfassend:
eine erste Korrektureinheit (70b), die das zweite grüne Bildsignal oder das zweite blaue Bildsignal derart korrigiert, dass der Signalpegel des zweiten grünen Bildsignals oder des zweiten blauen Bildsignals übereinstimmt mit einem Signalpegel des zweiten roten Bildsignals, falls der Signalpegel des zweiten grünen Bildsignals oder des zweiten blauen Bildsignals außerhalb eines spezifischen Bereichs liegt.

4. Endoskopsystem (10) nach Anspruch 2, weiterhin umfassend:
eine Signalpegel-Speichereinheit (72a), die einen Signalpegel des ersten grünen Bildsignals oder des ersten blauen Bildsignals speichert; und
eine zweite Korrektureinheit (72b), die das zweite grüne Bildsignal korrigiert, so dass der Signalpegel des zweiten grünen Bildsignals zu einem Signalpegel des ersten grünen Bildsignals wird, oder das zweite blaue Bildsignal derart korrigiert, dass ein Signalpegel des zweiten blauen Bildsignals zu einem Signalpegel des ersten blauen Bildsignals wird.

5. Endoskopsystem (10) nach Anspruch 4, weiterhin umfassend:
eine Anzeigeeinheit (18), die ein erstes Spezialbild auf der Grundlage des zweiten roten Bildsignals, des zweiten grünen Bildsignals und des zweiten blauen Bildsignals anzeigt, und ein zweites Spezialbild auf der Grundlage des zweiten roten Bildsignals, des zweiten grünen Bildsignals nach der Korrektur, und des zweiten blauen Bildsignals nach der Korrektur anzeigt.

6. Endoskopsystem (10) nach einem der Ansprüche 2 bis 5 weiterhin umfassend:
eine Durchschnittswert-Berechnungseinheit (63a), die einen Durchschnittswert der Pixelwerte des ersten roten Bildsignals, einen Durchschnittswert der Pixelwerte des ersten grünen Bildsignals und einen Durchschnittswert der Pixelwerte des ersten blauen Bildsignals auf der Grundlage des ersten roten Bildsignals, des ersten grünen Bildsignals und des ersten blauen Bildsignals berechnet,
wobei die Steuereinheit (21) die Lichtmenge von zumindest einer Farbe von dem roten Licht, dem grünen Licht und dem blauen Licht derart steuert, dass der Durchschnittswert der Pixelwerte des ersten grünen Bildsignals oder des ersten blauen Bildsignals übereinstimmt mit dem Durchschnittswert der Pixelwerte des ersten roten Bildsignals.

7. Endoskopsystem (10) nach einem der Ansprüche 1 oder 2, weiterhin umfassend:
eine Sollwert-Einstelleinheit, die einen ersten Sollwert aus dem Signalpegel des Bildsignals der ersten Farbe in dem ersten Farbbildsignal einstellt, wobei die Steuereinheit (21) das Lichtemissionsverhältnis des roten Lichts, des grünen Lichts und des blauen Lichts von dem ersten Lichtemissionsverhältnis zu einem dritten Lichtemissionsverhältnis ändert, anstatt das Lichtemissionsverhältnis des roten Lichts, des grünen Lichts und des blauen Lichts von dem ersten Lichtemissionsverhältnis zu dem zweiten Lichtemissionsverhältnis zu ändern, und die Lichtmenge von zumindest einer Farbe unter dem roten Licht, dem grünen Licht und dem blauen Licht derart steuert, dass die jeweiligen Signalpegel des Bildsignals der ersten Farbe, des Bildsignals der zweiten Farbe und des Bildsignals der dritten Farbe in dem ersten Farbbildsignal mit dem ersten Sollwert übereinstimmen.

8. Endoskopsystem (10) nach einem der Ansprüche 1 oder 2, weiterhin umfassend:
eine Sollwert-Einstelleinheit, die einen ersten Sollwert von dem Signalpegel des Bildsignals der ersten Farbe in dem ersten Farbbildsignal einstellt, einen zweiten Sollwert von dem Signalpegel des Bildsignals der zweiten Farbe in dem ersten Farbbildsignal einstellt, und einen dritten Sollwert von dem Signalpegel des Bildsignals der dritten Farbe in dem ersten Farbbildsignal einstellt,
wobei die Steuereinheit (21) das Lichtemissionsverhältnis des roten Lichts, des grünen Lichts und des blauen Lichts von dem ersten Lichtemissionsverhältnis zu einem vierten Lichtemissionsverhältnis ändert, anstatt das Lichtemissionsverhältnis des roten Lichts, des grünen Lichts und des blauen Lichts von dem ersten Lichtemissionsverhältnis zu dem zweiten Lichtemissionsverhältnis zu ändern, und die Lichtmenge von zumindest einer Farbe unter dem roten Licht, dem grünen Licht und dem blauen Licht derart steuert, dass der Signalpegel des Bildsignals der ersten Farbe in dem ersten Farbbildsignal übereinstimmt mit dem ersten Sollwert, der Signalpegel des Bildsignals der zweiten Farbe in dem ersten Farbbildsignal übereinstimmt mit dem zweiten Sollwert, und der Signalpegel des Bildsignals der dritten Farbe in dem ersten Farbbildsignal übereinstimmt mit dem dritten Sollwert.

## Revendications

1. Système d'endoscope (10), comprenant :
une unité de source lumineuse (14), laquelle émet une lumière rouge, une lumière verte, et une lumière bleue avec un premier rapport d'émission lumineuse ;
un capteur de prise d'images (48, 104), lequel image une cible d'observation dans un corps, et produit un signal d'image couleur incluant un signal d'image d'une première couleur, un signal d'image d'une deuxième couleur, et un signal d'image d'une troisième couleur, le capteur de prise d'images (48, 104) imageant la cible d'observation dans le corps éclairé par la lumière rouge, la lumière verte, et la lumière bleue avec le premier rapport d'émission lumineuse et produisant un premier signal d'image couleur, et
une unité de commande (21, 63) configurée pour modifier un rapport d'émission lumineuse entre la lumière rouge, la lumière verte, et la lumière bleue de l'unité de source lumineuse (14) du premier rapport d'émission lumineuse sur un deuxième rapport d'émission lumineuse, et pour commander la quantité de lumière d'au moins une couleur parmi la lumière rouge, la lumière verte, et la lumière bleue, de sorte qu'un niveau de signal du signal d'image de la deuxième couleur ou du signal d'image de la troisième couleur dans le premier signal d'image couleur coïncide avec un niveau de signal du signal d'image de la première couleur, et
dans lequel le capteur de prise d'images (48, 104) image la cible d'observation éclairée par la lumière rouge, la lumière verte, et la lumière bleue avec le deuxième rapport d'émission lumineuse, et produit un second signal d'image couleur.

2. Système d'endoscope (10) selon la revendication 1, dans lequel le signal d'image de la première couleur est un signal d'image rouge ; le signal d'image de la deuxième couleur est un signal d'image vert, et le signal d'image de la troisième couleur est un signal d'image bleu ;
le premier signal d'image couleur est un premier signal d'image rouge, un premier signal d'image vert, ou un premier signal d'image bleu, et
le second signal d'image couleur est un second signal d'image rouge, un second signal d'image vert, ou un second signal d'image bleu.

3. Système d'endoscope (10) selon la revendication 2, comprenant en outre :
une première unité de correction (70b), laquelle corrige le second signal d'image vert ou le second signal d'image bleu de sorte que le niveau de signal du second signal d'image vert ou du second signal d'image bleu coïncide avec un niveau de signal du second signal d'image rouge dans un cas où le niveau de signal du second signal d'image vert ou du second signal d'image bleu est hors d'une plage spécifique.

4. Système d'endoscope (10) selon la revendication 2, comprenant en outre :
une unité de stockage de niveau de signal (72a), laquelle stocke un niveau de signal du premier signal d'image vert ou du premier signal d'image bleu, et
une seconde unité de correction (72b), laquelle corrige le second signal d'image vert de sorte que le niveau de signal du second signal d'image vert devienne un niveau de signal du premier signal d'image vert, ou corrige le second signal d'image bleu, de sorte qu'un niveau de signal du second signal d'image bleu devienne un niveau de signal du premier signal d'image bleu.

5. Système d'endoscope (10) selon la revendication 4, comprenant en outre :
une unité d'affichage (18), laquelle affiche une première image spéciale sur la base du second signal d'image rouge, du second signal d'image vert, et du second signal d'image bleu, et affiche une seconde image spéciale sur la base du second signal d'image rouge, du second signal d'image vert après correction, et du second signal d'image bleu après correction.

6. Système d'endoscope (10) selon l'une quelconque des revendications 2 à 5, comprenant en outre :
une unité de calcul de valeur moyenne (63a), laquelle calcule une valeur moyenne des valeurs de pixel du premier signal d'image rouge, une valeur moyenne des valeurs de pixel du premier signal d'image vert, et une valeur moyenne des valeurs de pixel du premier signal d'image bleu sur la base du premier signal d'image rouge, du premier signal d'image vert, et du premier signal d'image bleu,
dans lequel l'unité de commande (21) commande la quantité de lumière d'au moins une couleur parmi la lumière rouge, la lumière verte, et la lumière bleue, de sorte que la valeur moyenne des valeurs de pixel du premier signal d'image vert ou du premier signal d'image bleu coïncide avec la valeur moyenne des valeurs de pixel du premier signal d'image rouge.

7. Système d'endoscope (10) selon la revendication 1 ou 2, comprenant en outre :
une unité de réglage de valeur cible, laquelle règle une première valeur cible à partir du niveau de signal du signal d'image de la première couleur dans le premier signal d'image couleur,
dans lequel l'unité de commande (21) modifie le rapport d'émission lumineuse entre la lumière rouge, la lumière verte et la lumière bleue du premier rapport d'émission lumineuse sur un troisième rapport d'émission lumineuse, au lieu de modifier le rapport d'émission lumineuse entre la lumière rouge, la lumière verte, et la lumière bleue du premier rapport d'émission lumineuse sur le deuxième rapport d'émission lumineuse, et commande la quantité de lumière d'au moins une lumière parmi la lumière rouge, la lumière verte, et la lumière bleue, de sorte que les niveaux de signal respectifs du signal d'image de la première couleur, du signal d'image de la deuxième couleur, et du signal d'image de la troisième couleur dans le premier signal d'image couleur coïncide avec la première valeur cible.

8. Système d'endoscope (10) selon la revendication 1 ou 2, comprenant en outre :
une unité de réglage de valeur cible, laquelle règle une première valeur cible à partir du niveau de signal du signal d'image de la première couleur dans le premier signal d'image couleur, règle une deuxième valeur cible à partir du niveau de signal du signal d'image de la deuxième couleur dans le premier signal d'image couleur, et règle une troisième valeur cible à partir du niveau de signal du signal d'image de la troisième couleur dans le premier signal d'image couleur,
dans lequel l'unité de commande (21) modifie le rapport d'émission lumineuse entre la lumière rouge, la lumière verte, et la lumière bleue du premier rapport d'émission lumineuse sur un quatrième rapport d'émission lumineuse, au lieu de modifier le rapport d'émission lumineuse entre la lumière rouge, la lumière verte, et la lumière bleue du premier rapport d'émission lumineuse sur le deuxième rapport d'émission lumineuse, et commande la quantité de lumière d'au moins une lumière parmi la lumière rouge, la lumière verte, et la lumière bleue, de sorte que le niveau de signal du signal d'image de la première couleur dans le premier signal d'image couleur coïncide avec la première valeur cible ; le niveau de signal du signal d'image de la deuxième couleur dans le premier signal d'image couleur coïncide avec la deuxième valeur cible, et le niveau de signal du signal d'image de la troisième couleur dans le premier signal d'image couleur coïncide avec la troisième valeur cible.
